(19) Europäisches Patentamt European Patent Office Office européen des brevets

(11) **EP 4 452 076 B1**

(12) **EUROPEAN PATENT SPECIFICATION**

(45) Date of publication and mention
of the grant of the patent:
**12.03.2025 Bulletin 2025/11**

(21) Application number: **22839775.8**

(22) Date of filing: **17.12.2022**

(51) International Patent Classification (IPC):
*A61B 6/03* (2006.01)    *A61B 6/00* (2024.01)
*A61B 6/42* (2024.01)    *A61B 6/58* (2024.01)

(52) Cooperative Patent Classification (CPC):
**A61B 6/035; A61B 6/4435; A61B 6/54;**
**A61B 6/547; A61B 6/58; A61B 6/582; A61B 6/586;**
A61B 6/4241; A61B 6/482

(86) International application number:
**PCT/EP2022/086519**

(87) International publication number:
**WO 2023/117819 (29.06.2023 Gazette 2023/26)**

(54) **METHOD FOR OPERATING A CT IMAGING SYSTEM**

VERFAHREN ZUM BETRIEB EINES CT-BILDGEBUNGSSYSTEMS

PROCÉDÉ DE FONCTIONNEMENT D'UN SYSTÈME D'IMAGERIE CT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **21.12.2021 EP 21216352**

(43) Date of publication of application:
**30.10.2024 Bulletin 2024/44**

(73) Proprietor: **Koninklijke Philips N.V.
5656 AG Eindhoven (NL)**

(72) Inventor: **PROKSA, Roland
5656 AG Eindhoven (NL)**

(74) Representative: **Philips Intellectual Property &
Standards
High Tech Campus 52
5656 AG Eindhoven (NL)**

(56) References cited:
US-A- 5 175 754        US-A1- 2009 045 859
US-B1- 6 574 301

**Description**

FIELD OF THE INVENTION

The invention provides a method for operating a CT imaging system, a data processing system, a system comprising the data processing system, a computer program product, and a computer readable medium.

BACKGROUND OF THE INVENTION

Data acquisition in many CT scanners uses angular triggering. An angular encoder generates trigger pulses at fixed gantry angles to trigger the integration periods (IP) of the detector. This concept allows for simplifying image reconstruction. Variations of the gantry speed impact the IP times, but not the angle. The time variations during the scan can be compensated by individual IP time measurements and data normalization.

For high resolution imaging, the accuracy of the angular trigger becomes important and a technical challenge. A mismatch of the actual angular trigger position in a scan and the assumed angle during reconstruction may cause image blurring and/or artefacts.

Thus, currently the accuracy of the angular trigger limits image quality of the reconstructed images. Sufficiently accurate angular triggers are a technical challenge and incur high costs.

US5175754A discloses the use of a phase-locked-loop using feedback techniques to fit a free running oscillator to a multiple of the frequency of an encoder signal to produce an appropriate delay in the encoder signal pulses as necessary to produce the acquisition signal pulses.

SUMMARY OF THE INVENTION

It is an object of the invention to allow for high image quality, particularly without incurring high costs.

The invention provides a method for operating a CT imaging system, a data processing system, a system comprising the data processing system, a computer program product, and a computer readable medium according to the independent claims. Preferred embodiments are laid down in the dependent claims.

The invention provides a method for operating a CT imaging system comprising a gantry having a detector and a rotary encoder attached to the gantry. The method comprises modelling, by means of an adaptive digital phase-locked loop, A-DPLL, a gantry rotation of the gantry, the A-DPLL configured to minimize the difference between an actual gantry angle $\alpha(t)_A$ and a modeled gantry angle $\alpha(t)_M$, and generating, for each of a plurality of predetermined values of the modeled gantry angle $\alpha(t)_M$, a trigger pulse for the detector. The actual gantry angle $\alpha(t)_A$ is obtained by detecting a gantry angle $\alpha(t)_D$ by means of the rotary encoder and adapting the detected gantry angle $\alpha(t)_D$ to account for a deviation of the actual rotary encoder characteristics from expected rotary encoder characteristics, the adapting being performed using an angular pattern $\beta_i$ of the rotary encoder.

A rotary encoder is inexpensive and reliable. However, the mechanical position and width accuracy of the slots limits the angular accuracy of the encoder. Due to the DPLL being adaptive, thereby allowing for taking into account the encoder inaccuracies, this type of inexpensive and inaccurate piece of equipment can be employed while still ensuring high image quality.

Accordingly, the method of the present disclosure allows for combining low component cost and high-resolution imaging. The method, for example, even allows for using inexpensive angular encoders such as slotted ring design without it being detrimental to accuracy.

Adaptive, in the present disclosure, may describe that the angular pattern of the rotary encoder is used for adapting the DPLL to take into account the actual characteristics of the encoder used to collect the data fed into the feedback loop, which may differ from expected characteristics.

In other words, the present disclosure allows for generating precise angular trigger pulses for high resolution CT imaging and high angular resolution by using an adaptive digital phase-locked loop which learns the angular pattern of the rotary encoder. The learned angular pattern may be used to correct trigger pulses for imaging.

As briefly mentioned above, the rotary encoder may, for example, be a slotted rotary encoder, in particular, comprise a slotted ring. A slotted ring rotary encoder comprises a metal strip having several slots and extending around the gantry, the slotted ring being part of the rotating portion of the rotary encoder or being part of the static portion of the rotary encoder. The rotary encoder may have an optical readout, particularly, comprise a slotted ring and an optical readout. In this case, the optical readout may be part of the static portion of the rotary encoder and the slotted ring may be part of the rotating portion of the rotary encoder or the optical readout may be part of the rotating portion of the rotary encoder and the slotted ring may be part of the static portion of the rotary encoder.

The method of the present disclosure may leverage that, although slotted encoders, in particular encoders having slotted rings, have a limited angular accuracy, they have a high repeatability from turn to turn. Since the slot position will not change, the angular error will stay constant per slot. This allows for reliable adaption, e.g., by means of a calibration procedure.

Operating a CT imaging system may comprise imaging steps, e.g., for example capturing CT images of a subject. Operating a CT imaging system may also comprise steps involved in preparing the CT imaging system for imaging, for example set-up and calibration steps, and/or steps following the imaging, for example processing of CT image data and analysis thereof. Operating a CT imaging system may also comprise any steps involved in controlling any component of the CT imaging system.

The CT imaging system may comprise, in addition to the detector, a source, for example an X-ray source. The gantry may comprise a rotatable frame, which may be configured to be rotated by means of a motor, and the detector of the CT imaging system may be attached to the frame. The source of the CT imaging system may also be attached to the frame, particularly such that the detector and the source are attached in an opposite arrangement and in such a manner that they rotate together with the rotatable frame.

The detector may be a photon counting detector or any other suitable CT imaging detector. The term detector may also entail a detector array, for example.

A rotary encoder generally may comprise a rotating portion and a static portion. A rotary encoder may be configured such that a rotation of the rotating portion with respect to the static portion can be quantified, particularly, as a rotation angle.

It is to be understood that the rotary encoder being attached to the gantry, in the present disclosure, means that the rotating portion of the rotary encoder is fixed to the gantry in such a manner that it rotates together with the gantry. Accordingly, the rotation of the gantry may correspond to the rotation of the rotating portion of the rotary encoder. The rotating portion may, for example, extend circumferentially around the gantry. The static portion is configured to remain static during operation of the rotary encoder.

Modelling a gantry rotation of the gantry may comprise, for example, using a model to predict a gantry angle, e.g., the modeled gantry angle $\alpha(t)_M$, the modelling aiming at a predicted gantry angle being as close as possible to an actual gantry angle $\alpha(t)_A$.

A digital phase-locked loop, DPLL, may be a feedback loop that allows for iteratively adjusting the model used for modelling the gantry angle $\alpha(t)_M$, so as to bring it closely to the actual gantry angle $\alpha(t)_A$. That is, the DPLL may iteratively improve the prediction of the gantry angle.

The trigger pulse for the detector may be any signal that causes the detector to start detection for obtaining a CT image. The capture or taking of each CT image may be triggered by a trigger pulse. As such, for each angle at which a CT image should be taken, a trigger pulse may be issued. Accordingly, the predetermined values of the modeled gantry angle $\alpha(t)_M$ may be values representing the angles at which an image should be taken.

Detecting a gantry angle $\alpha(t)_D$ by means of the rotary encoder comprises detecting the angle of the rotating portion of the rotary encoder relative to the fixed portion of the rotary encoder. As the rotating portion is fixed to the gantry, this directly reflects the gantry angle.

Encoder characteristics, in the present disclosure, may be characteristics reflecting the actual data that is output by the encoder in operation. The actual characteristics may be the characteristics actually witnessed/empirically determined for the rotary encoder after installation. Expected rotary encoder characteristics may be characteristics predicted by a model and/or provided by a manufacturer. The encoder characteristics may be expressed by an angular pattern. In particular, the angular pattern may, directly or indirectly, reflect the deviation between actual and expected rotary encoder characteristics.

The method may be a computer implemented method, in particular, all method steps may be carried out by a processing system.

In the present disclosure, the angular pattern $\beta_i$ may be accessed from a position look-up table, the position look-up table mapping each of a plurality of values of a gantry angle as detected by the encoder during a calibration procedure to a corresponding estimated actual value of the gantry angle as estimated during the calibration procedure.

For example, the look-up table may be stored in a computer-readable manner in a computer readable memory. Operating the imaging system may comprise that a data processing system receives at least one value of the detected gantry angle $\alpha(t)_D$, accesses the position look-up table and, for each detected gantry angle $\alpha(t)_D$, retrieves the estimated actual value mapped to the detected value, and uses the retrieved estimated actual values as the actual gantry angle $\alpha(t)_A$.

A look-up table, particularly when the size is not excessive, allows for fast and efficient retrieval of the angular pattern. Since generally the pattern of many rotary encoders, particularly slotted encoders, yields discrete values and the number of discrete values is limited by physical constraints of the rotary encoder, it is possible to store the angular pattern into a look-up table of reasonable size without loss of information on the angular pattern, thus allowing for reliable, fast, and efficient retrieval.

The method of the present disclosure may comprise determining the angular pattern $\beta_i$ of the rotary encoder by means of a/the calibration procedure, and, in particular, storing the angular pattern $\beta_i$ of the rotary encoder in a computer-readable memory and/or in a computer-readable manner, in particular into a/the position look-up table.

Determining the angular pattern by means of a calibration procedure will yield more accurate results than relying merely on manufacturer information and/or modelling. This is due to each rotary encoder, taken alone, deviating to some degree from models and/or manufacturer information and due to the CT imaging system having multiple interacting components that may influence measurement results of the rotary encoder and lead to such deviations. The calibration procedure may comprise an experimental component performed using the CT imaging system. This allows for accounting for such deviations. In addition, optionally, the calibration procedure may take into account manufacturer information and/or modelling of at least some of the components of the imaging system. In other words, the calibration may be based on empirical or semi-empirical methods. The calibration may make use of the fact that, while the encoder pattern may be irregular, i.e., deviate from a precise periodicity within one turn, its error also remains constant over time and from turn to turn, such that it repeats for each rotation.

Storing the angular pattern in a computer-readable manner may allow for directly using the data reflecting the angular pattern for the adapting step or steps. Regarding the position look-up table, reference is made to the above description.

The calibration procedure may comprise controlling the gantry to rotate and the rotary encoder to detect a plurality of angles per turn and determining slot times $T(1 \ldots N, 1 \ldots N_{Turn})$ for multiple turns, wherein $N$ is the number of slots of the rotary encoder and $N_{Turn}$ is the number of turns. The calibration procedure may further comprise normalizing the values of the slot times per turn and calculating slot angles $A(n, m)$, wherein

$$A(n,m) = \frac{2\pi T(n,m)}{\sum_{k=1}^{N} T(k,m)}.$$

The calibration procedure may further comprise averaging the values of the slot angles $A(n, m)$ of the multiple turns to obtain the angular pattern $\beta_i$ of the rotary encoder, wherein

$$\beta_i = \frac{\sum_{r=1}^{N_{Turn}} A(i,r)}{N_{Turn}},$$

wherein $\beta_i$ are values of the gantry angle.
in particular, wherein N is the number of slots, $i = 0 \ldots N - 1$.

The above is based on the assumption that rotation speed is constant during a turn. For giving the above features context, it is noted that an ideal encoder could be represented by the angular pattern:

$$\beta_i = \frac{2\pi i}{N-1}, i = 0 \ldots N - 1.$$

The method of the present disclosure accounts for the fact that any real encoder will differ from this ideal case. The above calibration is one possibility according to the present disclosure that allows for accounting for this difference.

The gantry may be controlled to be driven at maximal gantry speed while measuring the slot times. This allows for reducing the impact of speed variations during one turn. It is advantageous to avoid speed variations, as such variations will go into the angular pattern, e.g., the look-up table. Accordingly, the A-DPLL provides more accurate results when avoiding the speed variations during calibration, without taking any additional measures. For example, the A-DPLL, without taking additional measures will generally not be able to differentiate between speed variations and encoder irregularities, so avoiding speed variations improves accuracy.

In the present disclosure, obtaining the slot times $T(1 \ldots N, 1 \ldots N_{Turn})$ may comprise normalizing measured slot times to an estimated gantry speed during the measurement. Estimating the gantry speed to obtain the estimated gantry speed may be performed taking into account mechanical friction forces and/or mechanical forces due to gantry imbalances. Imbalances may be present when the center of mass is not in the rotation axis. Imbalances may modulate the rotation speed within each turn.

Alternatively or in addition to measuring the slot times while driving the gantry at maximal gantry speed, measuring the slot times may be performed during a period when the gantry is rotating without being driven by a motor, in particular, during deceleration of the gantry after driving, by means of the motor, the gantry at maximum gantry speed. In particular, during the period, the gantry may rotate without applying brakes. That is, measuring the slot times may be performed during a period when the gantry slows down freely.

Compared to the aspect of driving at maximum gantry speed during measuring the slot times, turning off the motor and letting the gantry slow down freely may allow for avoiding that the motor's characteristics distorts results of the calibration, thereby providing high accuracy.

For very high accuracy, two sets of slot times may be measured, one set while driving the gantry at maximal speed and one set during free deceleration of the gantry as outlined above. This may allow to compare or refine results obtained using one set of slot times with results obtained using the other set of slot times.

In the present disclosure, estimating the gantry speed may be performed taking into account mechanical forces due to gantry imbalances. The mechanical forces due to gantry imbalances may be modeled as $F_{Im} = c_0 \sin(\alpha + c_1)$ with $c_0$, $c_1$ being constants and $\alpha$ being actual gantry angle $\alpha(t)_A$. The impact of the mechanical forces due to gantry imbalances are derived from the energy loss, which is modeled as

$$\frac{dE}{dt} = d_0 \omega_t + d_1 \omega_t^2 + c_o \sin(\alpha + c_1).$$

The gantry speed $\omega_t$ may be estimated by fitting the free model parameters ($d_0$, $d_1$, $c_0$, $c_1$, $\omega_0$) to angles $\alpha(T_j)$ measured at times $T_j$ during the calibration.

Estimating the gantry speed in the manner proposed in the present disclosure and using it for obtaining the slot time allows for high accuracy, while avoiding that additional measurements are required for accounting for the characteristics of the gantry in the given setup and surroundings of the imaging system. Accordingly, high accuracy can be provided in an efficient manner.

The calibration procedure of the present disclosure may comprise CT image-based determination of the angular pattern. In particular, the calibration procedure may comprise analyzing CT projection data of a phantom obtained by the CT imaging system to detect a deviation of a shape of the phantom in the CT images and a shape of the phantom as expected when using a rotary encoder having the expected rotary encoder characteristics, and determining the angular pattern based on the deviation.

This example for determining angular pattern is driven by the effect that the calibration procedure will have on the CT imaging output. That is, it can be predicted what the output of a well-calibrated CT imaging system should be for a phantom of a predetermined shape. In the course of the calibration, if the output of the CT imaging system does not correspond to the predicted output, the angular pattern may be modified and it may then be determined whether applying the modified angular encoder pattern yields an output that corresponds to the predicted output or is within a predetermined interval around the predicted output. If this is the case, the angular pattern may be used for further operation of the CT imaging system. Otherwise, the angular pattern may be modified again, particularly repeatedly, until applying the modified angular pattern yields an output that corresponds to the predicted output or is within a predetermined interval around the predicted output. In other words, an initial angular pattern may be iteratively modified until it yields a predetermined output.

Projection data, in this context, may refer to raw data as obtained from the detector of the CT imaging system. Raw data may, for example, be 2D data for multi-row CT detectors.

An advantage of an image-based calibration is that it is aimed directly at the desired outcome of the calibration, i.e., accurate output from the CT imaging system, in particular, accurate CT images. An advantage of using non-image-based procedures as described further above may be that they can be performed without security measures that might be required for actually taking CT imaging with X-ray radiation.

Both, the image-based procedures and procedures that are not image-based, result in more accurate output. Optionally, the image-based procedures and procedures that are not image-based may be combined as a plausibility check and/or to increase accuracy even more.

The method of the present disclosure, particularly as part of a/the CT image-based calibration procedure, may comprise analyzing a plurality of CT projections, the CT projections obtained by means of the CT imaging system at a plurality of gantry angles and depicting a phantom, for example a single-wire phantom. The plurality of gantry angles, may, for example, cover at least one gantry rotation. The method may further comprise, for each of the CT projections, comparing an expected position of the phantom in the CT projection and an actual position of the phantom in the CT projection to determine a difference between the expected position and the actual position. The method may further comprise estimating, for each CT projection, an estimated gantry angle based on the difference between the expected position and the actual position. The method may further comprise determining the angular pattern based on differences between the estimated gantry angles and the corresponding gantry angles determined by the rotary encoder.

As an example for image-based calibration according to the present disclosure, the projection onto the detector array (i.e., the shadow) of a single wire phantom placed in the gantry oriented parallel to the z-axis (e.g., a long patient axis), when capturing a trans-axial 2D CT image, would be visualized by a spot. The position of the projection on the detector array depends on the wire position, the CT scanner geometry, and the actual gantry angle. For several gantry angles, the expected position for each projection may be determined and the expected positions may be compared with the measured positions. Differences indicate angular inaccuracies. The measured projection positions can be used to estimate the real gantry angle for each projection. The resulting data can be used for obtaining the angular pattern. The shadow position may optionally be estimated with subpixel-resolution with proper numerical methods.

A method of the present disclosure, particularly some or all of the above method steps, may be performed by a data processing system, also referred to as a computing system or computer. The method of the present disclosure may also comprise steps that are not performed by a data processing system, but that may optionally be controlled by a/the data processing system.

The method of the present disclosure may optionally comprise obtaining a/the CT image or images by means of the CT imaging system, in particular, by means of a photon counting CT imaging system, in particular by means of a low-dose CT scan. The method may comprise obtaining CT images during calibration and/or during operation after the calibration. A data processing system may be configured to control obtaining the CT images.

Alternatively or in addition, the method of the present disclosure may optionally comprise rotating the gantry by means of a motor, particularly based on the controlling the gantry to rotate as specified above. The method may comprise rotating the gantry during calibration and/or during operation after the calibration. A data processing system may control the rotating, particularly by sending control signals to the motor.

The invention also provides a data processing system for use in operating a CT imaging system comprising a gantry having a detector and a rotary encoder attached to the gantry. The data processing system is configured to model, by means of an adaptive digital phase-locked loop, A-DPLL, a gantry rotation of the gantry, the A-DPLL configured to minimize the difference between an actual gantry angle $\alpha(t)_A$ and a modeled gantry angle $\alpha(t)_M$. The data processing system is further configured to generate, for each of a plurality of predetermined values of the modeled gantry angle $\alpha(t)_M$, a

trigger pulse for the detector. The actual gantry angle $\alpha(t)_A$ is an angle obtained by detecting a gantry angle $\alpha(t)_D$ by means of the rotary encoder and adapting the detected gantry angle $\alpha(t)_D$ to account for a deviation of the actual rotary encoder characteristics from expected rotary encoder characteristics, the adapting being performed using an angular pattern $\beta_i$ of the rotary encoder. The data processing system may be configured to carry out and/or control any of the method steps of the present disclosure.

The invention also provides a system comprising the data processing system of the present disclosure and further comprising the CT imaging system comprising the gantry having the detector and the rotary encoder attached to the gantry, the CT imaging system configured to obtain a/the CT image or images. In particular, the CT imaging system may be a photon counting CT imaging system. The system may be configured to perform any of the methods of the present disclosure.

The invention also provides a computer program product comprising instructions which, when the program is executed by a computer, cause the computer to carry out one or more of the method steps described in the present disclosure.

The invention also provides a computer readable medium comprising instructions which, when executed by a computer, cause the computer to carry out one or more of the method steps described in the present disclosure.

The features and advantages outlined in the context of the method for operating the CT imaging system similarly apply to the data processing system, the system comprising the data processing system, the computer program product, and the computer readable medium of the present disclosure.

Further features, examples, and advantages will become apparent from the detailed description making reference to the accompanying drawings.

BRIEF DESCRIPTION OF THE DRAWINGS

Fig. 1 shows a flow diagram of a first method according to the present disclosure;
Fig. 2 shows a flow diagram of a second method according to the present disclosure;
Fig. 3 shows a schematic and not to scale illustration of a system according to the present disclosure; and
Fig. 4 shows a schematic illustration of the A-DPLL according to the present disclosure.

DETAILED DESCRIPTION OF EMBODIMENTS

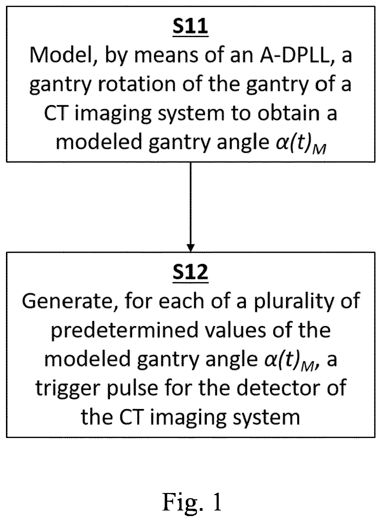

Fig. 1 shows a flow diagram of a method for operating a CT imaging system according to the present disclosure, the imaging system comprising a gantry having a detector and a rotary encoder attached to the gantry.

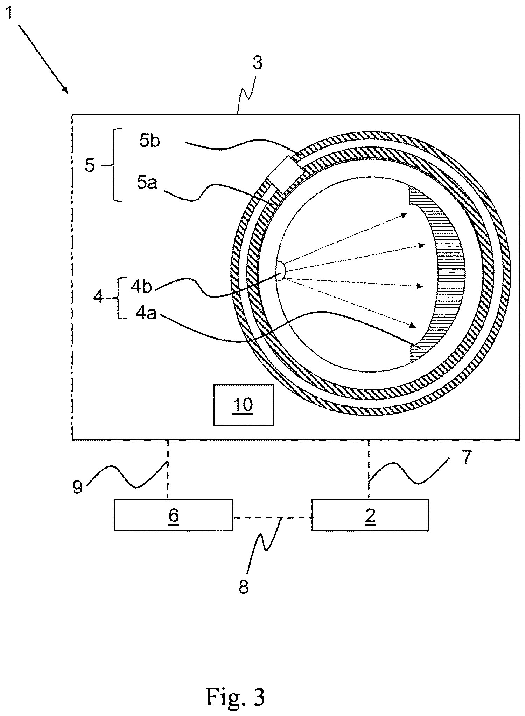

For example, the CT imaging system may be a CT imaging system as shown in and described below in the context of Fig. 3 or any other suitable system, e.g., according to the present disclosure.

The method shown in Fig. 1 comprises the step S11 of modelling, by means of an adaptive digital phase-locked loop, A-DPLL, a gantry rotation of the gantry, the A-DPLL configured to minimize the difference between an actual gantry angle $\alpha(t)_A$ and a modeled gantry angle $\alpha(t)_M$. The method shown in Fig. 1 comprises the step S12 of generating, for each of a plurality of predetermined values of the modeled gantry angle $\alpha(t)_M$, a trigger pulse for the detector.

According to the present disclosure, the actual gantry angle $\alpha(t)_A$ is obtained by detecting a gantry angle $\alpha(t)_D$ by means of the rotary encoder and adapting the detected gantry angle $\alpha(t)_D$ to account for a deviation of the actual rotary encoder characteristics from expected rotary encoder characteristics, the adapting being performed using an angular pattern $\beta_i$ of the rotary encoder.

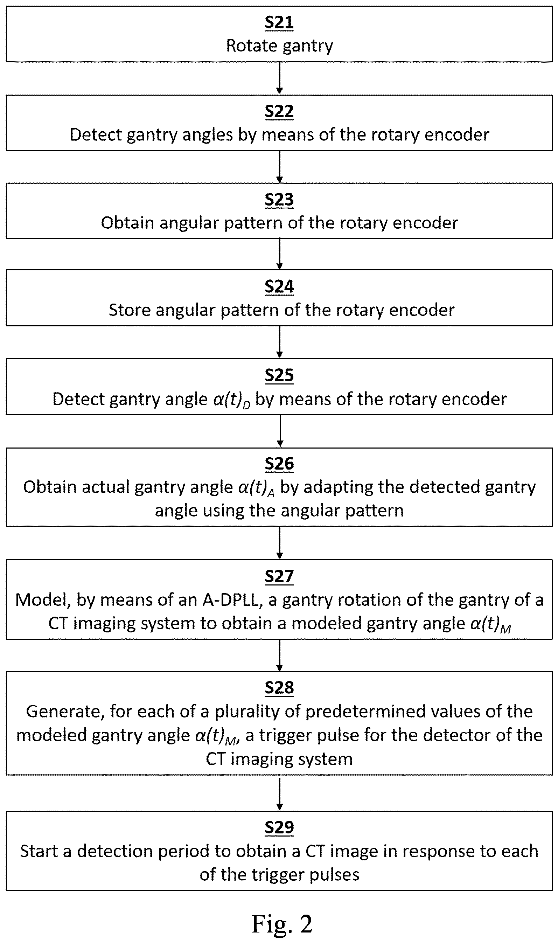

An exemplary, more detailed method according to the present disclosure, will be described in the following making reference to Fig. 2.

In steps S21 to S23, a calibration procedure is performed. In step S21, the gantry is rotated together with a rotatable portion of the rotary encoder. In step S22, a plurality of gantry angles is detected by means of the rotary encoder. In step S23, an angular pattern $\beta_i$ of the rotary encoder is obtained based on the detected gantry angles, for example as described above.

In step S24, the angular pattern $\beta_i$ of the rotary encoder is stored, in a computer readable manner, on a computer readable storage medium. For example, the angular pattern may be stored into a look-up table.

The above steps may, for example, be performed after setup or maintenance of the CT imaging system and prior to regular operation of the CT imaging system.

In step S25, which may be part of the regular operation of the CT imaging system, a gantry angle $\alpha(t)_D$ is detected by means of the rotary encoder.

In step S26, the actual gantry angle $\alpha(t)_A$ is obtained by adapting the detected gantry angle to account for a deviation of the actual rotary encoder characteristics from expected rotary encoder characteristics, the adapting being performed using the angular pattern $\beta_i$ of the rotary encoder. For example, the look-up table mentioned in the context S24 may be accessed to retrieve the angular pattern.

In step S27, the gantry rotation of the gantry is modeled by means of an adaptive digital phase-locked loop, A-DPLL on the basis of the actual gantry angle $\alpha(t)_A$ obtained in step S26, for example as described in the context of Fig.1, particularly step S11. Thus, for each detected gantry angle, and accordingly, each actual gantry angle, a modeled gantry angle $\alpha(t)_M$ is obtained.

In step S28, for each of a plurality of predetermined values of the modeled gantry angle $\alpha(t)_M$, a trigger pulse for the detector is generated, for example as described in the context of Fig.1, particularly step S12.

In step S29, in response to each of the trigger pulses, the CT imaging system may start a detection period to obtain a CT image.

Fig. 3 shows a schematic and not to scale illustration of a system 1 according to the present disclosure.

The system comprises a data processing system 2, which may be configured to perform one or more, in particular all, of the computer-implemented method steps of a method according to the present disclosure, particularly a method as described above in the context of Fig. 1 or Fig. 2.

The system 1 also comprises a CT imaging system 3, which comprises a CT gantry 4 having a detector 4a, for example a multi-row CT detector. The CT gantry may further comprise a source 4b, e.g., an X-ray source.

The CT imaging system further comprises a rotary encoder 5, which has a rotating portion 5a attached to the CT gantry in a positionally fixed manner. Thus, the rotating portion rotates together with the CT gantry. The rotary encoder also has a static portion 5b. The rotary encoder detects relative movement of the rotating portion 5a with respect to the static portion 5b, specifically, an angular change due to rotation of the rotating portion.

Optionally, the system may also comprise a display device 6, for example, configured to display CT images. Furthermore, optionally, the system may comprise data connections 7, 8, 9, for example wired or wireless data connections, connecting the data processing system and the CT imaging system, the data processing system and the display device, and the CT imaging system and the display device, respectively.

It is noted that some or all components of the system may be integrally formed, rather than being separately provided. For example, the data processing system and/or the display device may also be integrally formed with the CT imaging system and/or with each other.

A motor 10 for driving the CT gantry is also shown schematically as being comprised in the CT imaging system.

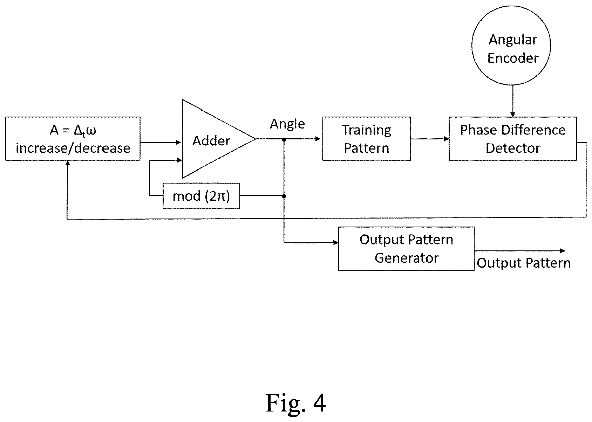

Fig 4. shows a schematic illustration of an A-DPLL according to the present invention. It is noted that in this context the angular pattern of the rotary encoder, e.g. as determined during a calibration procedure of the present disclosure, is used as a starting point for the training pattern shown in Fig. 4.

The commonly used terminology for PLLs uses the term *phase* to refer to the relative position of a periodic entity within in a cycle (e.g., the actual voltage in a sinusoidal electrical signal). In the present case, the gantry rotation is modeled as a periodic process and the term *angle* is used to define the relative position. The term *phase* is therefore equivalent to the gantry angle when applying PLL technology to periodic gantry rotation.

As can be seen from Fig. 4, measurement data may be provided by the angular encoder as input into a phase difference detector, which detects a phase difference with respect to data obtained from the training pattern. Based on the detected phase difference, the angular step for a time interval $A = \Delta_t\omega$ is increased or decreased and is used as input into an Adder, which outputs an angle/phase that is used as input for the training pattern. Moreover, the output angle can be input into an Ouput Pattern Generator that generates an output pattern to be used for triggering detection periods of a CT imaging system.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

LIST OF REFERENCE SIGNS:

S11 Model, by means of an A-DPLL, a gantry rotation of the gantry of a CT imaging system to obtain a modeled gantry angle $\alpha(t)_M$;

S12 Generate, for each of a plurality of predetermined values of the modeled gantry angle $\alpha(t)_M$, a trigger pulse for the detector of the CT imaging system;

S21 Rotate gantry;

S22 Detect gantry angles by means of the rotary encoder;

S23 Obtain angular pattern of the rotary encoder;

S24 Store angular pattern of the rotary encoder;

S25 Detect gantry angle $\alpha(t)_D$ by means of the rotary encoder;

S26 Obtain actual gantry angle $\alpha(t)_A$ by adapting the detected gantry angle using the angular pattern;

S27 Modelling, by means of an A-DPLL, a gantry rotation of the gantry of a CT imaging system to obtain a modeled gantry angle $\alpha(t)_M$;

S28 Generating, for each of a plurality of predetermined values of the modeled gantry angle $\alpha(t)_M$, a trigger pulse for the detector of the CT imaging system;

S29 Start a detection period to obtain a CT image in response to each of the trigger pulses;

System 1;

Data processing system 2;

CT imaging system 3;

CT gantry 4;

Detector 4a;

Source 4b;

Rotary encoder 5;
Rotating portion 5a
Static portion 5b;
Display device 6;
Data connections 7, 8, 9;
Motor 10

**Claims**

1. A method for operating a CT imaging system (3) comprising a gantry (4) having a detector (4a) and a rotary encoder (5) attached to the gantry (4), the method comprising:

   modelling, by means of an adaptive digital phase-locked loop, A-DPLL, a gantry rotation of the gantry (4), the A-DPLL configured to minimize the difference between an actual gantry angle $\alpha(t)_A$ and a modeled gantry angle $\alpha(t)_M$; and
   generating, for each of a plurality of predetermined values of the modeled gantry angle $\alpha(t)_M$, a trigger pulse for the detector (4a);
   wherein the actual gantry angle $\alpha(t)_A$ is obtained by detecting a gantry angle $\alpha(t)_D$ by means of the rotary encoder (5) and adapting the detected gantry angle $\alpha(t)_D$ to account for a deviation of the actual rotary encoder characteristics from expected rotary encoder characteristics, the adapting being performed using an angular pattern $\beta_i$ of the rotary encoder (5).

2. The method according to claim 1, wherein the angular pattern $\beta_i$ is accessed from a position look-up table, the position look-up table mapping each of a plurality of values of a gantry angle as detected by the rotary encoder (5) during a calibration procedure to a corresponding estimated actual value of the gantry angle as estimated during the calibration procedure.

3. The method according to any of the preceding claims, the method comprising determining the angular pattern $\beta_i$ of the rotary encoder (5) by means of a/the calibration procedure, and, in particular, storing the angular pattern $\beta_i$ of the rotary encoder (5) in a computer-readable memory, in particular into a/the position look-up table.

4. The method according to claim 3, wherein the calibration procedure comprises:

   controlling the gantry (4) to rotate and the rotary encoder (5) to detect a plurality of angles per turn;
   determining slot times $T(1 \ldots N , 1 \ldots N_{Turn})$ for multiple turns, wherein $N$ is the number of slots of the rotary encoder and $N_{Turn}$ is the number of turns;
normalizing the values of the slot times per turn and calculating slot angles $A(n, m)$, wherein

$$A(n, m) = \frac{2\pi T(n,m)}{\sum_{k=1}^{N} T(k,m)};$$

and

averaging the values of the slot angles $A(n, m)$ of the multiple turns to obtain the angular pattern $\beta_i$ of the rotary encoder (5), wherein

$$\beta_i = \frac{\sum_{r=1}^{N_{Turn}} A(i,r)}{N_{Turn}},$$

wherein $\beta_i$ are values of the gantry angle, in particular, wherein $N$ is the number of slots and $i = 0 \ldots N - 1$.

5. The method according to any of claim 4, wherein the gantry (4) is controlled to be driven at maximal gantry speed while measuring the slot times.

6. The method according to claim 4,

   wherein obtaining the slot times $T(1 \ldots N , 1 \ldots N_{Turn})$ comprises normalizing measured slot times to an estimated gantry speed during the measurement,
   wherein estimating the gantry speed is performed taking into account mechanical friction forces and/or mechanical forces due to gantry imbalances.

7. The method according to claim 6, wherein measuring the slot times is performed during a period when the gantry (4) is rotating without being driven by a motor (10), in particular, during deceleration of the gantry (4) after driving, by means of a/the motor (10), the gantry (4) at maximum gantry speed, in particular without applying brakes.

8. The method according to claim 6 or 7,

   wherein estimating the gantry speed is performed taking into account mechanical forces due to gantry imbalances,
   wherein the mechanical forces due to gantry imbalances are modeled as $F_{Im} = c_0 \sin(\alpha + c_1)$ with $c_0, c_1$ being constants and $\alpha$ being actual gantry angle $\alpha(t)_A$,
   wherein the impact of the mechanical forces due to gantry imbalances are derived from the energy loss, which is modeled as

$$\frac{dE}{dt} = d_0\omega_t + d_1\omega_t^2 + c_o\sin(\alpha + c_1),$$

and

wherein the gantry speed $\omega_t$ is estimated by fitting the free model parameters ($d_0$, $d_1$, $c_0$, $c_1$, $\omega_0$) to angles $\alpha(T_j)$ measured at times $T_j$ during the calibration.

9. The method according to any one of claims 3 to 8,

wherein the calibration procedure comprises CT image-based determination of the angular pattern,

in particular, wherein the calibration procedure comprises analyzing CT projection data of a phantom obtained by the CT imaging system (3) to detect a deviation of a shape of the phantom in the CT images and a shape of the phantom as expected when using a rotary encoder (5) having the expected rotary encoder characteristics, and determining the angular pattern based on the deviation.

10. The method according to any of the preceding claims, comprising, particularly as part of a/the calibration procedure,

analyzing a plurality of CT projections, the CT projections obtained by means of the CT imaging system (3) at a plurality of gantry angles and depicting a/the phantom, for example a/the single-wire phantom, in particular, wherein the plurality of gantry angles covers at least one gantry rotation;
for each of the CT projections, comparing an expected position of the phantom in the CT projection and an actual position of the phantom in the CT projection to determine a difference between the expected position and the actual position;
estimating, for each CT projection, an estimated gantry angle based on the difference between the expected position and the actual position; and
determining the angular pattern based on differences between the estimated gantry angles and the corresponding gantry angles determined by the rotary encoder.

11. The method according to any of the preceding claims and further comprising:

obtaining a/the CT image or CT images by means of the CT imaging system 83), in particular, by means of a photon counting CT imaging system, in particular by means of a low-dose CT scan; and/or

rotating the gantry (4) by means of a motor (10), particularly based on the controlling the gantry to rotate as specified in claim 4.

12. A data processing system (2) for use in operating a CT imaging system (3) comprising a gantry (4) having a detector (4a) and a rotary encoder (5) attached to the gantry, the data processing system configured to

model, by means of an adaptive digital phase-locked loop, A-DPLL, a gantry rotation of the gantry (4), the A-DPLL configured to minimize the difference between an actual gantry angle $\alpha(t)_A$ and a modeled gantry angle $\alpha(t)_M$; and
generate, for each of a plurality of predetermined values of the modeled gantry angle $\alpha(t)_M$, a trigger pulse for the detector (4a);
wherein the actual gantry angle $\alpha(t)_A$ is an angle obtained by detecting a gantry angle $\alpha(t)_D$ by means of the rotary encoder (5) and adapting the detected gantry angle $\alpha(t)_D$ to account for a deviation of the actual rotary encoder characteristics from expected rotary encoder characteristics, the adapting being performed using an angular pattern $\beta_i$ of the rotary encoder (5),
in particular, configured to carry out the method steps of any one of claims 1 to 10 and/or carry out and/or control the method steps of claim 11.

13. A system (1) comprising the data processing system (2) of claim 12 and further comprising:

the CT imaging system (3) comprising the gantry (4) having a detector (4a) and the rotary encoder (5) attached to the gantry (4), the CT imaging system (3) configured to obtain the CT images, in particular, wherein the CT imaging system (3) is a photon counting CT imaging system, in particular, wherein the system (1) is configured to perform the method of any one of claims 1 to 11.

14. A computer program product comprising instructions which, when the program is executed by a computer, cause the computer to carry out the method of any one of claims 1 to 10.

15. A computer readable medium comprising instructions which, when executed by a computer, cause the computer to carry out the method of any one of claims 1 to 10.

**Patentansprüche**

1. Verfahren zum Betreiben eines CT-Bildgebungssystems (3), das eine Gantry (4) umfasst, die einen Detektor (4a) und einen an der Gantry (4) angebrachten Drehgeber (5) aufweist, wobei das Verfahren umfasst:

Modellieren einer Gantry-Drehung der Gantry (4) mittels einer adaptiven digitalen Phasenregelschleife, A-DPLL, wobei die A-DPLL so konfiguriert ist, dass sie die Differenz zwischen einem tatsächlichen Gantry-Winkel $\alpha(t)_A$ und einem modellierten Gantry-Winkel $\alpha(t)_M$ minimiert; und
Erzeugen eines Auslöseimpulses für den Detektor (4a) für jeden einer Vielzahl von vorbestimmten Werten des modellierten Gantry-Winkels $\alpha(t)_M$;
wobei der tatsächliche Gantry-Winkel $\alpha(t)_A$ durch Erfassen eines Gantry-Winkels $\alpha(t)_D$ mittels des Drehgebers (5) und Anpassen des erfassten Gantry-Winkels $\alpha(t)_D$, um eine Abweichung der tatsächlichen Drehgebereigenschaften von erwarteten Drehgebereigenschaften zu berücksichtigen, erhalten wird, wobei das Anpassen unter Verwendung eines Winkelmusters $\beta_i$ des Drehgebers (5) durchgeführt wird.

2. Verfahren nach Anspruch 1, wobei aus einer Positionsnachschlagetabelle auf das Winkelmuster $\beta_i$ zugegriffen wird, wobei die Positionsnachschlagetabelle jeden einer Vielzahl von Werten eines Gantry-Winkels, wie sie vom Drehgeber (5) während eines Kalibrierungsvorgangs erfasst werden, auf einen entsprechenden geschätzten tatsächlichen Wert des Gantry-Winkels, wie er während des Kalibrierungsvorgangs geschätzt wird, abbildet.

3. Verfahren nach einem der vorstehenden Ansprüche, wobei das Verfahren Bestimmen des Winkelmusters $\beta_i$ des Drehgebers (5) mittels eines/des Kalibrierungsvorgangs und insbesondere das Speichern des Winkelmusters $\beta_i$ des Drehgebers (5) in einem computerlesbaren Speicher, insbesondere in einer/der Positionsnachschlagetabelle, umfasst.

4. Verfahren nach Anspruch 3, wobei der Kalibrierungsvorgang umfasst:

Steuern der Gantry (4) so, dass sie sich dreht, und des Drehgebers (5) so, dass er eine Vielzahl von Winkeln pro Umdrehung erfasst;
Bestimmen von Schlitzzeiten $T(1 \dots N, 1 \dots N_{Umdrehung})$ für mehrere Umdrehungen, wobei $N$ die Anzahl der Schlitze des Drehgebers ist und $N_{Umdrehung}$ die Anzahl der Umdrehungen ist;
Normalisieren der Werte der Schlitzzeiten pro Umdrehung und Berechnen von Schlitzwinkeln $A(n, m)$, wobei

$$A(n, m) = \frac{2\pi T(n, m)}{\sum_{k=1}^{N} T(k, m)};$$

und
Mitteln der Werte der Schlitzwinkel $A(n, m)$ der mehreren Umdrehungen, um das Winkelmuster $\beta_i$ des Drehgebers (5) zu erhalten, wobei

$$\beta_i = \frac{\sum_{r=1}^{N_{Umdrehung}} A(i, r)}{N_{Umdrehung}},$$

wobei $\beta_i$ Werte des Gantry-Winkels sind, insbesondere wobei $N$ die Anzahl der Slots ist und $i = 0 \dots N - 1$.

5. Verfahren nach einem von Anspruch 4, wobei die Gantry (4) so gesteuert wird, dass sie während des Messens der Schlitzzeiten mit maximaler Gantry-Geschwindigkeit angetrieben wird.

6. Verfahren nach Anspruch 4,

wobei das Erhalten der Schlitzzeiten $T(1 \dots N, 1 \dots N_{Umdrehung})$ das Normalisieren von gemessenen Schlitzzeiten auf eine geschätzte Gantry-Geschwindigkeit während der Messung umfasst,
wobei das Schätzen der Gantry-Geschwindigkeit unter Berücksichtigung von mechanischen Reibungskräften und/oder mechanischen Kräften aufgrund von Gantry-Unwuchten durchgeführt wird.

7. Verfahren nach Anspruch 6, wobei Messen der Schlitzzeiten während eines Zeitraums durchgeführt wird, in dem sich die Gantry (4) dreht, ohne von einem Motor (10) angetrieben zu werden, insbesondere während der Verlangsamung der Gantry (4) nach dem Antreiben der Gantry (4) mittels eines/des Motors (10) mit maximaler Gantry-Geschwindigkeit, insbesondere ohne Anwenden von Bremsen.

8. Verfahren nach Anspruch 6 oder 7,

wobei das Schätzen der Gantry-Geschwindigkeit unter Berücksichtigung von mechanischen Kräften aufgrund von Gantry-Unwuchten durchgeführt wird,
wobei die mechanischen Kräfte aufgrund von Gantry-Unwuchten als $F_{lm} = c_0 \sin(\alpha + c_1)$ modelliert werden, wobei $c_0$, $c_1$ Konstanten sind und $\alpha$ der tatsächliche Gantry-Winkel $\alpha(t)_A$ ist, wobei die Auswirkung der mechanischen Kräfte aufgrund von Gantry-Unwuchten aus dem Energieverlust abgeleitet werden, der modelliert wird als

$$\frac{dE}{dt} = d_0 \omega_t + d_1 \omega_t^2 + c_o \sin(\alpha + c_1),$$

und
wobei die Gantry-Geschwindigkeit $\omega_t$ durch Anpassen der freien Modellparameter ($d_0$, $d_1$, $c_0$, $c_1$, $\omega_0$) an die während der Kalibrierung zu den Zeitpunkten $T_j$ gemessenen Winkel $\alpha(T_j)$ geschätzt wird.

9. Verfahren nach einem der Ansprüche 3 bis 8,

wobei der Kalibrierungsvorgang eine CT-Bild-basierte Bestimmung des Winkelmusters umfasst,
insbesondere wobei der Kalibrierungsvorgang das Analysieren von CT-Projektionsdaten eines Phantoms, die durch das CT-Bildgebungssystem (3) erhalten werden, um eine Abweichung einer Form des Phantoms in den CT-Bildern und einer Form des Phantoms, wie sie erwartet wird, wenn ein Drehgeber (5), der die erwarteten Drehgebereigenschaften aufweist, verwendet wird, zu erfassen, und das Bestimmen des Winkelmusters auf Basis der Abweichung umfasst.

10. Verfahren nach einem der vorstehenden Ansprüche, das insbesondere als Teil eines/des Kalibrierungsvorgangs umfasst

Analysieren einer Vielzahl von CT-Projektionen, wobei die CT-Projektionen mittels des CT-Bildgebungssystems (3) bei einer Vielzahl von Gantry-Winkeln erhalten werden und ein/das Phantom, zum Beispiel ein/das Eindrahtphantom, abbilden, insbesondere wobei die Vielzahl von Gantry-Winkeln mindestens eine Gantry-Drehung abdeckt;
Vergleichen einer erwarteten Position des Phantoms in der CT-Projektion und einer tatsächlichen Position des Phantoms in der CT-Projektion für jede der CT-Projektionen, um eine Differenz zwischen der erwarteten Position und der tatsächlichen Position zu bestimmen;
Schätzen eines geschätzten Gantry-Winkels für jede CT-Projektion auf Basis der Differenz zwischen der erwarteten Position und der tatsächlichen Position; und
Bestimmen des Winkelmusters auf Basis von Differenzen zwischen den geschätzten Gantry-Winkeln und den entsprechenden Gantry-Winkeln, die durch den Drehgeber bestimmt wurden.

11. Verfahren nach einem der vorstehenden Ansprüche und weiter umfassend:

Erhalten eines/des CT-Bildes oder von CT-Bildern mittels des CT-Bildgebungssystems (3), insbesondere mittels eines Photonenzähl-CT-Bildgebungssystems, insbesondere mittels eines Niedrigdosis-CT-Scans; und/oder
Drehen der Gantry (4) mittels eines Motors (10), insbesondere auf Basis der Steuerung der Gantry so, dass sich diese wie in Anspruch 4 spezifiziert dreht.

12. Datenverarbeitungssystem (2) zur Verwendung beim Betrieb eines CT-Bildgebungssystems (3), das eine Gantry (4) umfasst, die einen Detektor (4a) und einen an der Gantry angebrachten Drehgeber (5) aufweist, wobei das Datenverarbeitungssystem so konfiguriert ist, dass es

mittels einer adaptiven digitalen Phasenregelschleife, A-DPLL, eine Gantry-Drehung der Gantry (4) modelliert, wobei die A-DPLL so konfiguriert ist, dass sie die Differenz zwischen einem tatsächlichen Gantry-Winkel $\alpha(t)_A$ und einem modellierten Gantry-Winkel $\alpha(t)_M$ minimiert; und
für jeden einer Vielzahl von vorbestimmten Werten des modellierten Gantry-Winkels $\alpha(t)_M$ einen Auslöseimpuls für den Detektor (4a) erzeugt;
wobei der tatsächliche Gantry-Winkel $\alpha(t)_A$ ein Winkel ist, der durch Erfassen eines Gantry-Winkels $\alpha(t)_D$ mittels des Drehgebers (5) und Anpassen des erfassten Gantry-Winkels $\alpha(t)_D$, um eine Abweichung der tatsächlichen Drehgebereigenschaften von erwarteten Drehgebereigenschaften zu berücksichtigen, erhalten wird, wobei das Anpassen unter Verwendung eines Winkelmusters $\beta_i$ des Drehgebers (5) durchgeführt wird,
insbesondere so konfiguriert ist, dass es die Verfahrensschritte nach einem der Ansprüche 1 bis 10 ausführt und/oder die Verfahrensschritte nach Anspruch 11 ausführt und/oder steuert.

13. System (1), das das Datenverarbeitungssystem (2) nach Anspruch 12 umfasst, und weiter umfassend:

das CT-Bildgebungssystem (3), das die Gantry (4) umfasst, die einen Detektor (4a) und den an der Gantry (4) angebrachten Drehgeber (5) aufweist, wobei das CT-Bildgebungssystem (3) so konfiguriert ist, dass es die CT-Bilder erhält, insbesondere wobei das CT-Bildgebungssystem (3) ein Photonenzähl-CT-Bildgebungssystem ist,
insbesondere wobei das System (1) so konfiguriert ist, dass es das Verfahren nach einem der Ansprüche 1 bis 11 durchführt.

14. Computerprogrammprodukt, das Anweisungen umfasst, die, wenn das Programm von einem Computer ausgeführt wird, bewirken, dass der Computer das Verfahren nach einem der Ansprüche 1 bis 10 ausführt.

15. Computerlesbares Medium, das Anweisungen umfasst, die, wenn sie von einem Computer ausgeführt werden, bewirken, dass der Computer das Verfahren nach einem der Ansprüche 1 bis 10 ausführt.

**Revendications**

1. Procédé de fonctionnement d'un système d'imagerie CT (3) comprenant un portique (4) présentant un détecteur (4a) et un codeur rotatif (5) fixé au portique (4), le procédé comprenant :

   la modélisation, au moyen d'une boucle à verrouillage de phase numérique adaptative, A-DPLL, d'une rotation de portique du portique (4), l'A-DPLL étant configurée pour réduire au minimum la différence entre un angle de portique réel $\alpha(t)_A$ et un angle de portique modélisé $\alpha(t)_M$ ; et
   la génération, pour chacune d'une pluralité de valeurs prédéterminées de l'angle de portique modélisé $\alpha(t)_M$, d'une impulsion de déclenchement pour le détecteur (4a) ;
   dans lequel l'angle de portique réel $\alpha(t)_A$ est obtenu en détectant un angle de portique $\alpha(t)_D$ au moyen du codeur rotatif (5) et en adaptant l'angle de portique détecté $\alpha(t)_D$ pour tenir compte d'un écart des caractéristiques de codeur rotatif réelles par rapport aux caractéristiques de codeur rotatif attendues, l'adaptation étant effectuée à l'aide d'un motif angulaire $\beta_i$ du codeur rotatif (5).

2. Procédé selon la revendication 1, dans lequel le motif angulaire $\beta_i$ est accessible à partir d'une table de consultation de position, la table de consultation de position mappant chacune d'une pluralité de valeurs d'un angle de portique tel que détecté par le codeur rotatif (5) pendant une procédure d'étalonnage à une valeur réelle estimée correspondante de l'angle de portique tel qu'estimé pendant la procédure d'étalonnage.

3. Procédé selon l'une quelconque des revendications précédentes, le procédé comprenant la détermination du motif angulaire $\beta_i$ du codeur rotatif (5) au moyen d'une/de la procédure d'étalonnage, et, en particulier, le stockage du motif angulaire $\beta_i$ du codeur rotatif (5) dans une mémoire lisible par ordinateur, en particulier dans une/la table de consultation de position.

4. Procédé selon la revendication 3, dans lequel la procédure d'étalonnage comprend :

   la commande de la rotation du portique (4) et de la détection par le codeur rotatif (5) d'une pluralité d'angles par tour ;
   la détermination de temps de fente $T(1 ... N, 1... N_{Tour})$ pour plusieurs tours, dans lequel $N$ est le nombre de fentes du codeur rotatif et $N_{Tour}$ est le nombre de tours ;
   la normalisation des valeurs des temps de fente par tour et le calcul des angles de fente $A(n, m)$, dans lequel

   $$A(n,m) = \frac{2\pi T(n,m)}{\sum_{k=1}^{N} T(k,m)} \; ;$$

   et
   le calcul de la moyenne des valeurs des angles de fente $A(n, m)$ des multiples tours pour obtenir le motif angulaire $\beta_i$ du codeur rotatif (5), dans lequel

   $$\beta_i = \frac{\sum_{r=1}^{N_{Tour}} A(i,r)}{N_{Tour}},$$

   dans lequel $\beta_i$ sont les valeurs de l'angle de portique,
   en particulier, dans lequel $N$ est le nombre de fentes et $i = 0 ... N - 1$.

5. Procédé selon l'une quelconque de la revendication 4, dans lequel le portique (4) est commandé pour être entraîné à une vitesse de portique maximale tout en mesurant les temps de fente.

6. Procédé selon la revendication 4,

   dans lequel l'obtention des temps de fente $T(1 ... N, 1 ... N_{Tour})$ comprend la normalisation des temps de fente mesurés à une vitesse de portique estimée pendant la mesure,
   dans lequel l'estimation de la vitesse de portique est effectuée en tenant compte des forces de frottement mécaniques et/ou des forces mécaniques dues aux déséquilibres du portique.

7. Procédé selon la revendication 6, dans lequel la mesure des temps de fente est effectuée pendant une période où le portique (4) tourne sans être entraîné par un moteur (10), en particulier, pendant la décélération du portique (4) après avoir entraîné, au moyen d'un/du moteur (10), le portique (4) à vitesse de portique maximale, en particulier sans appliquer de freins.

8. Procédé selon la revendication 6 ou 7,

dans lequel l'estimation de la vitesse de portique est effectuée en tenant compte des forces mécaniques dues aux déséquilibres du portique, dans lequel les forces mécaniques dues aux déséquilibres du portique sont modélisées par $F_{lm} = c_0 \sin(\alpha + c_1)$ avec $c_0$, $c_1$ étant des constantes et $\alpha$ étant l'angle de portique réel $\alpha(t)_A$, dans lequel l'impact des forces mécaniques dues aux déséquilibres du portique est déduit de la perte d'énergie, qui est modélisée par

$$\frac{dE}{dt} = d_0 \omega_t + d_1 \omega_t^2 + c_o \sin(\alpha + c_1),$$

et

dans lequel la vitesse de portique $\omega_t$ est estimée en adaptant les paramètres de modèle libre ($d_0$, $d_1$, $c_0$, $c_1$, $\omega_0$) aux angles $\alpha(T_j)$ mesurés aux instants $T_j$ pendant l'étalonnage.

**9.** Procédé selon l'une quelconque des revendications 3 à 8,

dans lequel la procédure d'étalonnage comprend la détermination du motif angulaire basée sur une image CT,
en particulier, dans lequel la procédure d'étalonnage comprend l'analyse de données de projection CT d'un fantôme obtenu par le système d'imagerie CT (3) pour détecter un écart d'une forme du fantôme dans les images CT et une forme du fantôme tel qu'attendu lors de l'utilisation d'un codeur rotatif (5) présentant les caractéristiques de codeur rotatif attendues, et la détermination du motif angulaire sur la base de l'écart.

**10.** Procédé selon l'une quelconque des revendications précédentes, comprenant, notamment dans le cadre d'une/de la procédure d'étalonnage,

l'analyse d'une pluralité de projections CT, les projections CT étant obtenues au moyen du système d'imagerie CT (3) à une pluralité d'angles de portique et représentant un/le fantôme, par exemple un/le fantôme monofilaire, en particulier, dans lequel la pluralité d'angles de portique couvrent au moins une rotation de portique ;
pour chacune des projections CT, la comparaison d'une position attendue du fantôme dans la projection CT et d'une position réelle du fantôme dans la projection CT pour déterminer une différence entre la position attendue et la position réelle ;
l'estimation, pour chaque projection CT, d'un angle de portique estimé sur la base de la différence entre la position attendue et la position réelle ; et
la détermination du modèle angulaire sur la base de différences entre les angles de portique estimés et les angles de portique correspondants déterminés par le codeur rotatif.

**11.** Procédé selon l'une quelconque des revendications précédentes et comprenant en outre :

l'obtention d'une/de l'image CT ou des images CT au moyen du système d'imagerie CT (3), en particulier, au moyen d'un système d'imagerie CT à comptage de photons, en particulier au moyen d'un scanner CT à faible dose ; et/ou
la rotation du portique (4) au moyen d'un moteur (10), en particulier sur la base de la commande de la rotation du portique telle que spécifiée dans la revendication 4.

**12.** Système de traitement de données (2) destiné à être utilisé dans le fonctionnement d'un système d'imagerie CT (3) comprenant un portique (4) présentant un détecteur (4a) et un codeur rotatif (5) fixé au portique, le système de traitement de données étant configuré pour

modéliser, au moyen d'une boucle à verrouillage de phase numérique adaptative, A-DPLL, une rotation de portique du portique (4), l'A-DPLL étant configurée pour réduire au minimum la différence entre un angle de portique réel $\alpha(t)_A$ et un angle de portique modélisé $\alpha(t)_M$ ; et
générer, pour chacune d'une pluralité de valeurs prédéterminées de l'angle de portique modélisé $\alpha(t)_M$, une impulsion de déclenchement pour le détecteur (4a) ;
dans lequel l'angle de portique réel $\alpha(t)_A$ est un angle obtenu en détectant un angle de portique $\alpha(t)_D$ au moyen du codeur rotatif (5) et en adaptant l'angle de portique détecté $\alpha(t)_D$ pour tenir compte d'un écart des caractéristiques de codeur rotatif réelles par rapport aux caractéristiques de codeur rotatif attendues, l'adaptation étant effectuée à l'aide d'un motif angulaire $\beta_i$ du codeur rotatif (5),
en particulier, configuré pour mettre en œuvre les étapes de procédé selon l'une quelconque des revendications 1 à 10 et/ou mettre en œuvre et/ou commander les étapes de procédé selon la revendication 11.

**13.** Système (1) comprenant le système de traitement de données (2) selon la revendication 12 et comprenant en outre :

le système d'imagerie CT (3) comprenant le portique (4) présentant un détecteur (4a) et le codeur rotatif (5) fixé au portique (4), le système d'imagerie CT (3) étant configuré pour obtenir les images CT, en particulier, dans lequel le système d'imagerie CT (3) est un système d'imagerie CT à comptage de photons,
en particulier, dans lequel le système (1) est configuré pour mettre en œuvre le procédé selon l'une quelconque des revendications 1 à 11.

**14.** Produit de programme informatique comprenant des instructions qui, lorsque le programme est exécuté par un ordinateur, amènent l'ordinateur à mettre en œuvre le procédé selon l'une quelconque des revendications 1 à 10.

**15.** Support lisible par ordinateur comprenant des instructions qui, lorsqu'elles sont exécutées par un ordinateur, amènent l'ordinateur à effectuer le procédé selon l'une quelconque des revendications 1 à 10.

**S11**
Model, by means of an A-DPLL, a gantry rotation of the gantry of a CT imaging system to obtain a modeled gantry angle $\alpha(t)_M$

**S12**
Generate, for each of a plurality of predetermined values of the modeled gantry angle $\alpha(t)_M$, a trigger pulse for the detector of the CT imaging system

Fig. 1

**S21**
Rotate gantry

**S22**
Detect gantry angles by means of the rotary encoder

**S23**
Obtain angular pattern of the rotary encoder

**S24**
Store angular pattern of the rotary encoder

**S25**
Detect gantry angle $\alpha(t)_D$ by means of the rotary encoder

**S26**
Obtain actual gantry angle $\alpha(t)_A$ by adapting the detected gantry angle using the angular pattern

**S27**
Model, by means of an A-DPLL, a gantry rotation of the gantry of a CT imaging system to obtain a modeled gantry angle $\alpha(t)_M$

**S28**
Generate, for each of a plurality of predetermined values of the modeled gantry angle $\alpha(t)_M$, a trigger pulse for the detector of the CT imaging system

**S29**
Start a detection period to obtain a CT image in response to each of the trigger pulses

Fig. 2

Fig. 3

Fig. 4

**EP 4 452 076 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5175754 A